(19) Europäisches Patentamt
European Patent Office
Office européen des brevets

(11) EP 2 115 499 B1

(12) **EUROPEAN PATENT SPECIFICATION**

(45) Date of publication and mention of the grant of the patent:
**11.11.2015 Bulletin 2015/46**

(21) Application number: **08841458.6**

(22) Date of filing: **22.02.2008**

(51) Int Cl.:
***G02B 1/04*** *(2006.01)* ***B29D 11/00*** *(2006.01)*

(86) International application number:
**PCT/US2008/054641**

(87) International publication number:
**WO 2009/055082 (30.04.2009 Gazette 2009/18)**

# (54) METHOD FOR IMPARTING HYDROGEL CONTACT LENSES WITH DESIRED PROPERTIES

VERFAHREN ZUR AUSSTATTUNG VON HYDROGEL-KONTAKTLINSEN MIT DEN GEWÜNSCHTEN EIGENSCHAFTEN

PROCÉDÉ POUR CONFÉRER DES PROPRIÉTÉS SOUHAITÉES À DES LENTILLES DE CONTACT EN HYDROGEL

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **26.02.2007 US 891572 P**

(43) Date of publication of application:
**11.11.2009 Bulletin 2009/46**

(73) Proprietor: **Novartis AG**
**4056 Basel (CH)**

(72) Inventors:
- **QIU, Yongxing**
  **Duluth**
  **Georgia 30097 (US)**
- **VOGT, Jürgen**
  **CH-4112 Flüh (CH)**
- **LALLY, John Martin**
  **Benbrook, TX 76126 (US)**

(74) Representative: **Bohest AG**
**Holbeinstrasse 36-38**
**4051 Basel (CH)**

(56) References cited:
**EP-A- 1 754 494**
**US-A1- 2005 226 931**
**US-A1- 2007 037 898**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 115 499 B1

## Description

The present invention generally relates to a method for imparting hydrogel contact lenses, in particular, silicone hydrogel contact lenses, one or more desirable properties, such as, antimicrobial property and/or lubricity. In addition, the present invention provides hydrogel contact lenses made according to method of the invention.

## BACKGROUND OF THE INVENTION

Currently, a greater effort in the contact lens industry has been devoted to develop technologies for producing contact lenses which have minimal adverse effects on corneal health and wearer's comfort.

One of the active areas in the corneal health aspect is to develop antimicrobial contact lenses, because one or more microorganisms during contact lens wear, storage and handling may adhere/proliferate to form a colony on the ocular surface and thereby may cause infection or other deleterious effects on the ocular health of the eye in which the lens is used. Various approaches have been proposed, such as, for example, Chalkley et al.'s publication in Am. J. Ophthalmology 1966, 61:866-869 (contact lenses with germicidal agents incorporated therein); U.S. Patent No. 4,472,327 (contact lenses with antimicrobial agents which may be added to the monomer before polymerization and locked into the polymeric structure of the lenses); U.S. Patent Nos. 5,358,688 and 5,536,861 and European Patent Application Publication No. EP0604369 (contact lenses containing quaternary ammonium group containing organosilicone polymers); European Patent Application Publication No. EP0947856A2 (contact lenses containing a quaternary phosphonium group-containing polymer); U.S. Patent No. 5,515,117 (contact lenses comprising polymeric materials and antimicrobial compound); U.S. Patent No. 5,213,801 (contact lenses including an antimicrobial ceramics containing at least one metal selected from Ag, Cu and Zn); U.S. Patent No. 5,328,954 (contact lenses with coatings composed of a wide variety of antimicrobial agents; a commonly owned co-pending U.S. Patent Application Publication No. 2005/0013842A1 (silver nanoparticles incorporated in extended-wear contact lenses to impart to the contact lenses an effective antimicrobial capability over a long period of time); co-pending U.S. Patent Application Publication Nos. 2005/0058844A1 and 2005/0008676A1 (contact lenses with an antimicrobial LbL coating thereon), European Patent Application No. EP1754494A2 (antimicrobial lenses displaying extended efficacy, obtained by immersing the lens in a solution containing silver ions), U.S. Patent Application Publication No. 2007/0037898 (the polymerizable fluid used for making the antimicrobial contact lenses comprise stabilized anti-microbiol silver nanoparticles). In spite of the forgoing efforts, there are no commercially available contact lenses, especially extended-wear contact lenses, which exhibit antimicrobial activities over a long period of time. Therefore, there is still need for methods of making antimicrobial hydrogel contact lenses.

Although various approaches have been proposed for enhancing lens wearer's comfort, there is still a need for a cost-efficient and simple method for making hydrogel contact lenses which can provide a good comfort for lens wearers.

## SUMMARY OF THE INVENTION

The invention, in one aspect, provides a method of making hydrogen contact lenses according to claim 1.

The invention, in another aspect, provides a method of making hydrogel contact lenses according to claim 9, comprising the steps of: polymerizing a lens-forming formulation to form a hydrogel contact lens; and hydrating the polymerized hydrogel contact lens in an aqueous solution containing silver nanoparticles and a lubricant or wetting agent as defined in claims 1 to 8.

## DETAILED DESCRIPTION OF EMBODIMENTS OF THE INVENTION

Reference now will be made in detail to the embodiments of the invention. Features illustrated or described as part of one embodiment, can be used on another embodiment to yield a still further embodiment.

Unless defined otherwise, all technical and scientific terms used herein have the same meaning as commonly understood by one of ordinary skill in the art to which this invention belongs. Generally, the nomenclature used herein and the laboratory procedures are well known and commonly employed in the art. Conventional methods are used for these procedures, such as those provided in the art and various general references. Where a term is provided in the singular, the inventors also contemplate the plural of that term. The nomenclature used herein and the laboratory procedures described below are those well-known and commonly employed in the art.

"Contact Lens" refers to a structure that can be placed on or within a wearer's eye. A contact lens can correct, improve, or alter a user's eyesight, but that need not be the case. A contact lens can be of any appropriate material known in the art or later developed, and can be a soft lens, a hard lens, or a hybrid lens. A "silicone hydrogel contact lens" refers to a contact lens comprising a silicone hydrogel material.

A "hydrogel" or a "hydrogel material" refers to a polymeric material which can absorb at least 10 percent by weight of water when it is fully hydrated.

A "silicone hydrogel" refers to a silicone-containing hydrogel obtained by copolymerization of a polymerizable composition comprising at least one silicone-containing monomer or at least one silicone-containing macromer or at least one crosslinkable silicone-containing prepolymer.

"Hydrophilic," as used herein, describes a material or portion thereof that will more readily associate with water than with lipids.

A "monomer" means a low molecular weight compound that can be polymerized actinically or thermally. Low molecular weight typically means average molecular weights less than 700 Daltons. In accordance with the invention, a monomer can be a vinylic monomer or a compound comprising two thiol groups. A compound with two thiol groups can participate in thiol-ene step-growth radical polymerization with a monomer with vinyl group to form a polymer. Step-growth radical polymerization can be used in making contact lenses, as described in a commonly-owned copending U.S. Patent Application No. 60/869,812 filed Dec. 13, 2006 (entitled "PRODUCTION OF OPHTHALMIC DEVICES BASED ON PHOTO-INDUCED STEP GROWTH POLYMERIZATION".

A "vinylic monomer", as used herein, refers to a low molecular weight compound that has an ethylenically unsaturated group and can be polymerized actinically or thermally. Low molecular weight typically means average molecular weights less than 700 Daltons.

The term "olefinically unsaturated group" or "ethylenically unsaturated group" is employed herein in a broad sense and is intended to encompass any groups containing at least one >C=C< group. Exemplary ethylenically unsaturated groups include without limitation acryloyl, methacryloyl, allyl, vinyl, styrenyl, or other C=C containing groups.

As used herein, "actinically" in reference to curing or polymerizing of a polymerizable composition or material means that the curing (e.g., crosslinked and/or polymerized) is performed by actinic irradiation, such as, for example, UV irradiation, ionized radiation (e.g. gamma ray or X-ray irradiation), microwave irradiation, and the like. Thermal curing or actinic curing methods are well-known to a person skilled in the art.

A "hydrophilic monomer" refers to a monomer which can be polymerized actinically or thermally to form a polymer that is water-soluble or can absorb at least 10 percent by weight water.

A "hydrophobic monomer", as used herein, refers to a vinylic monomer which is polymerized actinically or thermally to form a polymer that is insoluble in water and can absorb less than 10 percent by weight water.

A "macromer" refers to a medium and high molecular weight compound which can be polymerized and/or crosslinked actinically or thermally. Medium and high molecular weight typically means average molecular weights greater than 700 Daltons. In accordance with the invention, a macromer comprises one or more ethylenically unsaturated groups and/or one or more thiol groups, which can participate in free radical chain growth polymerization or thiol-ene step-growth radical polymerization. Preferably, a macromer contains ethylenically unsaturated groups and can be polymerized actinically or thermally.

A "prepolymer" refers to a starting polymer which contains crosslinkable groups and can be cured (e.g., crosslinked and/or polymerized) actinically or thermally to obtain a crosslinked and/or polymerized polymer having a molecular weight much higher than the starting polymer. In accordance with the invention, a prepolymer comprises one or more ethylenically unsaturated groups and/or one or more thiol groups, which can participate in free radical chain growth polymerization or thiol-ene step-growth radical polymerization.

A "silicone-containing prepolymer" refers to a prepolymer which contains silicone and can be crosslinked upon actinic radiation or thermally to obtain a crosslinked polymer having a molecular weight much higher than the starting polymer.

"Molecular weight" of a polymeric material (including monomeric or macromeric materials), as used herein, refers to the number-average molecular weight unless otherwise specifically noted or unless testing conditions indicate otherwise.

A "polymer" means a material formed by polymerizing/crosslinking one or more monomers.

An "initiator" is intended to describe a chemical that initiates free radical reaction and can be a photoinitiator or thermal initiator.

A "photoinitiator" refers to a chemical that initiates radical crosslinking/polymerizing reaction by the use of light. Suitable photoinitiators include, without limitation, benzoin methyl ether, diethoxyacetophenone, a benzoylphosphine oxide, 1-hydroxycyclohexyl phenyl ketone, Darocure® types, and Irgacure® types, preferably Darocure® 1173, and Irgacure® 2959.

A "thermal initiator" refers to a chemical that initiates radical crosslinking/polymerizing reaction by the use of heat energy. Examples of suitable thermal initiators include, but are not limited to, 2,2'-azobis (2,4-dimethylpentanenitrile), 2,2'-azobis (2-methylpropanenitrile), 2,2'-azobis (2-methylbutanenitrile), peroxides such as benzoyl peroxide, and the like. Preferably, the thermal initiator is 2,2'-azobis(isobutyronitrile) (AIBN).

A "spatial limitation of actinic radiation" refers to an act or process in which energy radiation in the form of rays is directed by, for example, a mask or screen or combinations thereof, to impinge, in a spatially restricted manner, onto

an area having a well defined peripheral boundary. For example, a spatial limitation of UV radiation can be achieved by using a mask or screen that has a transparent or open region (unmasked region) surrounded by a UV impermeable region (masked region), as schematically illustrated in Figs 1-9 of U.S. Patent No. 6,627,124 (herein incorporated by reference). The unmasked region has a well defined peripheral boundary with the unmasked region. The energy used for the crosslinking is radiation energy, especially UV radiation, gamma radiation, electron radiation or thermal radiation, the radiation energy preferably being in the form of a substantially parallel beam in order on the one hand to achieve good restriction and on the other hand efficient use of the energy.

"Visibility tinting" in reference to a lens means dying (or coloring) of a lens to enable the user to easily locate a lens in a clear solution within a lens storage, disinfecting or cleaning container. It is well known in the art that a dye and/or a pigment can be used in visibility tinting a lens.

"Dye" means a substance that is soluble in a solvent and that is used to impart color. Dyes are typically translucent and absorb but do not scatter light. Any suitable biocompatible dye can be used in the present invention.

A "pigment" means a powdered substance that is suspended in a liquid in which it is insoluble. A pigment can be a fluorescent pigment, phosphorescent pigment, pearlescent pigment, or conventional pigment. While any suitable pigment may be employed, it is presently preferred that the pigment be heat resistant, non-toxic and insoluble in aqueous solutions.

"Surface modification", as used herein, means that an article has been treated in a surface treatment process (or a surface modification process), in which, by means of contact with a vapor or liquid, and/or by means of application of an energy source (1) a coating is applied to the surface of an article, (2) chemical species are adsorbed onto the surface of an article, (3) the chemical nature (e.g., electrostatic charge) of chemical groups on the surface of an article are altered, or (4) the surface properties of an article are otherwise modified. Exemplary surface treatment processes include, but are not limited to, a surface treatment by energy (e.g., a plasma, a static electrical charge, irradiation, or other energy source), chemical treatments, the grafting of hydrophilic monomers or macromers onto the surface of an article, and layer-by-layer deposition of polymeric materials. A preferred class of surface treatment processes are plasma processes, in which an ionized gas is applied to the surface of an article. Plasma gases and processing conditions are described more fully in U.S. Patent Nos. 4,312,575 and 4,632,844. The plasma gas is preferably a mixture of lower alkanes and nitrogen, oxygen or an inert gas.

"LbL coating", as used herein, refers to a coating that is not covalently attached to a contact lens or a mold half and is obtained through a layer-by-layer ("LbL") deposition of polyionic (or charged) and/or non-charged materials on the lens or mold half. An LbL coasting can be composed of one or more layers, preferably one or more bilayers. Formation of an LbL coating on a contact lens or mold half may be accomplished in a number of ways, for example, as described in U.S. Patent Nos. 6,451,871, 6,719,929, 6,793,973, 6,811,805 and 6,896,926.

An "average contact angle " refers to a water contact angle (measured by Sessile Drop method), which is obtained by averaging measurements of at least 3 individual contact lenses.

As used herein, "increased surface hydrophilicity" or "increased hydrophilicity" in reference to a hydrated contact lens means that a hydrated contact lens, which is obtained by hydrating a dry contact lens or an unhydrated contact lens in a hydrating solution of the invention, has a reduced averaged contact angle relative to a control contact lens which is obtained by hydrating a dry contact lens or an unhydrated contact lens in water or a buffered saline, wherein both hydrated and control contact lenses are made of the same core material.

An "antimicrobial contact lens", as used herein, refers to a contact lens that exhibit at least a 5-fold reduction (≥80% inhibition), preferably at least a 1-log reduction (≥90% inhibition), more preferably at least a 2-log reduction (≥99% inhibition), of viable microorganisms.

An "antimicrobial agent", as used herein, refers to a chemical that is capable of decreasing or eliminating or inhibiting the growth of microorganisms such as that term is known in the art.

"Ag-nanoparticles" refer to particles which are made essentially of silver metal and have a size of about 1 micrometer or less. Silver in the nanoparticles can be present in one or more of its oxidation states, such as $Ag^0$, $Ag^{1+}$, and $Ag^{2+}$. It is understood that Ag-nanoparticles may undergo aggregation in a fluid composition and the apparent size of Ag-nanoparticles may be several micrometers when analyzed by particle size analyzer without turning on the ultrasonication function of the particle size analyzer (e.g., particle size analyzer Horiba LA-920).

"Stabilized Ag-nanoparticles" refer to Ag-nanoparticles which are formed in the presence of a stabilizer and are stabilized by the stabilizer. Stabilized Ag-nanoparticles can be either positively charged or negatively charged or neutral, largely depending on a material (or so-called stabilizer) which is present in a solution for preparing Ag-nanoparticles and can stabilize the resultant Ag-nanoparticles. A stabilizer can be any known suitable material. Exemplary stabilizers include, without limitation, positively charged polyionic materials, negatively charged polyionic materials, polymers, surfactants, salicylic acid, alcohols and the like.

*"in-situ"* formation of Ag-nanoparticles refers to a process in which Ag-nanoparticles are formed directly in a hydrating solution for hydrating a hydrogel contact lens without further purification and separation of the formed Ag-nanoparticles. The formation of Ag-nanoparticles can be confirmed by UV spectroscopy with absorption peaks around

a wavelength of about 460 nm or smaller, a characteristic of Ag-nanoparticles.

"Chloride-treated Ag-nanoparticles" refer to Ag-nanoparticles obtained according to a process in which after Ag-nanoparticles are formed in a dispersion, chloride is added in the dispersion containing the formed Ag-nanoparticles therein so as to reduce substantially the characteristic yellowish color of untreated Ag-nanoparticles.

"Lyophilizing" refers to a freeze-drying process in which the solvent is removed substantival.

An "unhydrated" hydrogel contact lens refers to a hydrogel contact lens either which still contains the solvent of a lens-forming formulation from which the lens is made or which has been subjected in an extraction process in which unpolymerized polymerizable components are removed from the lens by using a solvent, typically a water-soluble or miscible organic solvent or a mixture of water and an organic solvent.

The present invention is generally directed to simple and cost-effective methods for making hydrogel contact lenses with desired properties, such as antimicrobial property and enhanced lens wearer's comfort. The present invention is partly based on the discovery that by simply contacting a dry or unhydrated hydrogel contact lens with an aqueous solution (or hydrating solution) containing silver nanoparticles and a lubricant or wetting agent, one can make a hydrogel contact lens with antimicrobial property and enhanced lens wearer's comfort.

Although the inventors do not wish to be bound by any particular theory, it is believed that when hydrating a dry or unhydrated hydrogel contact lens with an aqueous solution (or hydrating solution) containing silver nanoparticles and a lubricant and/or wetting agent, silver nanoparticles and lubricant or wetting agent can be adsorbed onto the hydrogel contact lens to form a coating and/or can be adsorbed/entrapped in the polymer matrix of the hydrogel contact lens to become a leachable (releasable) lubricant or wetting agent which can be leached (or released) slowly over an extended period of time.

Most hydrogel contact lenses which are cast-molded according to conventional cast molding technologies either require extraction or hydration or both. For example, manufacturing process for silicone hydrogel contact lenses, Focus® Night & Day™ and O2OPTIX™ (from CIBA Vision), involves, *inter alia,* plasma treatment to render the surfaces of silicone hydrogel lenses hydrophilic and wettable. The plasma coating process generally requires drying the lenses before subjecting lenses to the plasma treatment. Plasma-treated lenses then need to be hydrated before packaging. By incorporating silver nanoparticles and a lubricant or wetting agent in a hydrating solution, one can achieve three goals in one simple process: hydrating lenses, imparting lenses antimicrobial property, and increased lubricity and wettability of lenses. It is believed that where a silicone hydrogel lens having a plasma coating, silver nanoparticles and lubricants or wetting agents are likely to be adsorbed onto the plasma coating to impart the lens the antimicrobial property and increased lubricity and wettability. There is no need for separate additional steps.

By using a process of the invention, one can incorporate silver nanoparticles and lubricants/wetting agents onto and/or into a hydrogel contact lens, one can impart antimicrobial capability and enhanced comfort without significantly adverse effects on the desired bulk properties of the lens, such as oxygen permeability, ion or water permeability. Silver nanoparticles can slowly release silver ion into their surrounding fluid (e.g., tear) to provide the contact lens antimicrobial property. The lubricant or wetting agent adsorbed on the surface of the contact lens and entrapped in the lens can be slowly released to provide the contact lens increased lubricity and wettability (characterized by an increased surface hydrophilicity) and thereby enhance wearer's comfort. Further, the lubricant or wetting agent on the surface of the contact lens and in the lens may have different releasing speeds and can be released at different time scale. Where silver nanoparticles are stabilized by a lubricant or wetting agent, the stabilized silver nanoparticles can release slowly silver ion and lubricant or wetting agent over extended period of time to provide prolonged antimicrobial property and lens wearer's comfort.

The invention, in one aspect, provides a method of making hydrogel contact lenses, comprising the steps of: hydrating a dry hydrogel contact lens in a hydrating solution, wherein the hydrating solution comprises silver nanoparticles and a lubricant or wetting agent, wherein the silver nanoparticles and the lubricant or wetting agent are adsorbed onto and/or entrapped in the hydrogel contact lens during hydrating of the dry hydrogel contact lens in the hydrating solution.

Any known suitable methods can be used in the preparation of Ag-nanoparticles. For example, silver ions or silver salts can be reduced by means of a reducing agent (e.g., $NaBH_4$, ascorbic acid, citrate, or the like) or of heating or UV irradiation in a solution in the presence of a stabilizer to form Ag-nanoparticles. A person skilled in the art will know how to choose a suitable known method for preparing Ag-nanoparticles. It is understood that Agnanoparticles can be lyophilized (freeze-dried) and then can be re-dispersed in an aqueous hydrating solution.

Any known suitable soluble silver salts can be used in the present invention. Preferably, silver nitrate is used.

Where Ag-nanoparticles are prepared *in situ* in a hydrating solution (e.g., without further purification steps), a biocompatible reducing agent is preferably used. Examples of biocompatible reducing agents includes without limitation ascorbic acid and biocompatible salts thereof, and biocompatible salts of citrate.

Ag-nanoparticles are prepared in the presence of one or more stabilizers so as to obtain stabilized Ag-nanoparticles. A "stabilizer" refers to a material which is present in a solution for preparing the nano-particles and can stabilize the resultant nano-particles. A small amount of a stabilizer present in a hydrating solution can improve greatly the stability of the a hydrating dispersion. In accordance with the present invention, a stabilizer is a polyanionic material, a polycationic

material, a polyvinyl alcohol (PVA), a polyvinylpyrrolidone (PVP) or a copolymer of n-vinylpyrrolidone with one ore more vinylic monomers.

A polycationic material used in the present invention can generally include any material known in the art to have a plurality of positively charged groups along a polymer chain. For instance, suitable examples of such polycationic materials can include, but are not limited to, poly(allylamine hydrochloride) (PAH), poly(ethylenimine) (PEI), copolymers of vinylpyrrolidone with one or more vinylic monomer having a quaternary ammonium groups (e.g., vinylpyrrolidone/dimethylaminomethylmethacrylate (DMAEMA) copolymers), poly(pyridinium acetylene), and poly(vinylbenzyltriamethylamine) (PVBT).

A polyanionic material used in the present invention can generally include any material known in the art to have a plurality of negatively charged groups along a polymer chain. For example, suitable polyanionic materials can include, but are not limited to, polymethacrylic acid (PMA), polyacrylic acid (PAA), copolymers of acrylic acid with one or more vinylic monomers, copolymers of methacrylic acid with one or more vinylic monomers, poly(4-styrenesulfonic acid) (PSS), sodium poly(styrene sulfonate) (SPS) and poly(sodium styrene sulfonate) (PSSS).

A preferred stabilizer is polyacrylic acid (PAA), poly(ethylenimine) (PEI), a copolymer of acrylic acid with one or more vinylic monomers, acrylic acid/vinylpyrrolidone copolymers, a copolymer of methacrylic acid with one or more vinylic monomers, methacrylic acid/vinylpyrrolidone copolymers, polyvinylpyrrolidone (PVP) of a molecular weight of up to 1,500,000, a copolymer (of a molecular weight of up to 1,500,000) of vinylpyrrolidone with one or more vinylic monomer, a polyionic material having amino groups and/or sulfur-containing groups or mixture thereof.

Exemplary sulfur-containing groups include, without limitation, thiol, sulfonyl, sulfonic acid, alkyl sulfide, alkyl disulfide, substituted or unsubstituted phenyldisulfide, thiophenyl, thiourea, thioether, thiazolyl, thiazolinyl, and the like.

The amount of a stabilizer in a hydrating solution or dispersion is less than 1% percent by weight, preferably less than 0.5% by weight, more preferably less than 0.1% by weight.

It should be pointed out that where a stabilizer is a -COOH-containing polymer (e.g., PAA), an amino-containing polycationic polymer, or a sulfur-containing polyionic polymer, the concentration of the stabilizer should be at a level below which silver ions can be reduced into Ag-nanoparticles.

The hydrating solution or dispersion containing stabilized Ag-nanoparticles is preferably treated with chloride. With such simple chloride treatment, one converts the free silver ions to silver chloride and the characteristic yellowish color of silver nanoparticle in the hydrating solution can be minimized or eliminated.

In accordance with the invention, "treating of the Ag-nanoparticles-containing polymerizable dispersion with chloride" refers to introducing of chloride ions into the hydrating solution or dispersion.

In one embodiment, treating of the Ag-nanoparticles-containing hydrating solution or dispersion with chloride can be performed by: (1) adding chloride salt, such as NaCl in solid form, directly into the dispersion; (2) mixing thoroughly the mixture for a period of time long enough to substantially reduce the characteristic yellowish color of Ag-nanoparticles in the dispersion.

In another embodiment, the chloride treatment can be carried out by: (1) adding a NaCl solution or hydrochloride into the Ag-nanoparticles-containing hydrating solution or dispersion and (2) mixing thoroughly the mixture for a period of time long enough to substantially reduce characteristic yellowish color of Ag-nanoparticles in the hydrating solution or dispersion.

Examples of lubricants include without limitation mucin-like materials and hydrophilic polymers. Exemplary mucin-like materials include without limitation polyglycolic acid, polylactides, collagen, hyaluronic acid, and gelatin.

Exemplary hydrophilic polymers include, but are not limited to, polyvinyl alcohols (PVAs), polyamides, polyimides, polylactone, a homopolymer of a vinyl lactam, a copolymer of at least one vinyl lactam in the presence or in the absence of one or more hydrophilic vinylic comonomers, alkylated polyvinylpyrrolidones, a homopolymer of acrylamide or methacrylamide, a copolymer of acrylamide or methacrylamide with one or more hydrophilic vinylic monomers, polyethylene oxide, (i.e., polyethylene glycol (PEG)), a polyoxyethylene derivative, poly-N-N-dimethylacrylamide, polyacrylic acid, poly-2-ethyl oxazoline, tieparin polysaccharides, polysaccharides, and mixtures thereof.

Examples of N-vinyl lactams include N-vinyl-2-pyrrolidone, N-vinyl-2-piperidone, N-vinyl-2-caprolactam, N-vinyl-3-methyl-2-pyrrolidone, N-vinyl-3-methyl-2-piperidone, N-vinyl-3-methyl-2-caprolactam, N-vinyl-4-methyl-2-pyrrolidone, N-vinyl-4-methyl-2-caprolactam, N-vinyl-5-methyl-2-pyrrolidone, N-vinyl-5-methyl-2-piperidone, N-vinyl-5,5-dimethyl-2-pyrrolidone, N-vinyl-3,3,5-trimethyl-2-pyrrolidone, N-vinyl-5-methyl-5-ethyl-2-pyrrolidone, N-vinyl-3,4,5-trimethyl-3-ethyl-2-pyrrolidone, N-vinyl-6-methyl-2-piperidone, N-vinyl-6-ethyl-2-piperidone, N-vinyl-3,5-dimethyl-2-piperidone, N-vinyl-4,4-dimethyl-2-piperidone, N-vinyl-7-methyl-2-caprolactam, N-vinyl-7-ethyl-2-caprolactam, N-vinyl-3,5-dimethyl-2-caprolactam, N-vinyl-4,6-dimethyl-2-caprolactam, and N-vinyl-3,5,7-trimethyl-2-caprolactam.

The number-average molecular weight $M_n$ of the hydrophilic polymer is, for example, greater than 10,000, or greater than 20,000, than that of the matrix forming material. For example, when the matrix forming material is a water-soluble prepolymer having an average molecular weight $M_n$ of from 12,000 to 25,000, the average molecular weight $M_n$ of the hydrophilic polymer is, for example, from 25,000 to 100000, from 30,000 to 75,000, or from 35,000 to 70,000.

Examples of polyvinylpyrrolidone (PVP) include without limitation those polymers characterized by molecular

weight grades of K-15, K-30, K-60, K-90, K-120, and the likes.

Examples of copolymers of n-vinylpyrrolidone with one ore more vinylic monomers includes without limitation vinylpyrrolidone/vinylacetate copolymers, vinylpyrrolidone/dimethylaminoethylmethacrylate copolymers (e.g., Copolymer 845, Copolymer 937, Copolymer 958 from ISP Corporation), vinylpyrrolidone/vinylcaprolactam/dimethyl-aminoethylmethacrylate copolymer.

Examples of alkylated pyrrolidones includes without limitation the family of GANEX® Alkylated pyrrolidone from ISP Corporation.

In a preferred embodiment, the hydrating solution comprises a mixture of polyvinylpyrrolidones with different molecular weights. By having different molecular weights (generally characterized by K-value), PVP can be released at different timescales, thereby providing to the resultant contact lens a controlled time release of PVP over an extended period of time (e.g., over at least about 6 hours). In such embodiment, the hydrating solution preferably comprises at least one high molecular weight PVP (i.e., 60-K or higher) and at least one low molecular weight PVP (i.e., lower than 60-K).

In another preferred embodiment, the hydrating solution comprises at least one polyvinylpyrrolidone and at least one copolymer of vinylpyrrolidone with one or more vinylic monomers. By using a mixture of PVP and PVP copolymer, one may have a controlled releasing profile of lubricant/wetting agents.

In another preferred embodiment, the hydrating solution comprises at least one polyvinylpyrrolidone and at least one alkylated polyvinylpyrrolidone. By using a mixture of PVP and alkylated polyvinylpyrrolidone, one may have a controlled releasing profile of lubricant/wetting agents.

A suitable polyoxyethylene derivative is, for example, n-alkylphenyl polyoxyethylene ether, n-alkyl polyoxyethylene ether (e.g., TRITON®), polyglycol ether surfactant (TERGITOL®), polyoxyethylenesorbitan (e.g., TWEEN®), polyoxyethylated glycol monoether (e.g., BRIJ®, polyoxylethylene 9 lauryl ether, polyoxylethylene 10 ether, polyoxylethylene 10 tridecyl ether), or a block copolymer of ethylene oxide and propylene oxide.

Examples of block copolymers of ethylene oxide and propylene oxide include without limitation poloxamers and poloxamines, which are available, for example, under the trade name PLURONIC®, PLURONIC-R®, TETRONIC®, TETRONIC-R® or PLURADOT®. Poloxamers are triblock copolymers with the structure PEO-PPO-PEO (where "PEO" is poly(ethylene oxide) and "PPO" is poly(propylene oxide).

A considerable number of poloxamers is known, differing merely in the molecular weight and in the PEO/PPO ratio; Examples of poloxamers include 101, 105, 108, 122, 123, 124, 181, 182, 183, 184, 185, 188, 212, 215, 217, 231, 234, 235, 237, 238, 282, 284, 288, 331, 333, 334, 335, 338, 401, 402, 403 and 407. The order of polyoxyethylene and polyoxypropylene blocks can be reversed creating block copolymers with the structure PPO-PEO-PPO, which are known as PLURONIC-R® polymers.

Poloxamines are polymers with the structure $(PEO\text{-}PPO)_2\text{-}N\text{-}(CH_2)_2\text{-}N\text{-}(PPO\text{-}PEO)_2$ that are available with different molecular weights and PEO/PPO ratios. Again, the order of polyoxyethylene and polyoxypropylene blocks can be reversed creating block copolymers with the structure $(PPO\text{-}PEO)_2\text{-}N\text{-}(CH_2)_2\text{-}N\text{-}(PEO\text{-}PPO)_2$, which are known as TETRONIC-R® polymers.

Polyoxypropylene-polyoxyethylene block copolymers can also be designed with hydrophilic blocks comprising a random mix of ethylene oxide and propylene oxide repeating units. To maintain the hydrophilic character of the block, ethylene oxide will predominate. Similarly, the hydrophobic block can be a mixture of ethylene oxide and propylene oxide repeating units. Such block copolymers are available under the trade name PLURADOT®.

In accordance with the invention, the step of hydrating a dry hydrogel contact lens is performed at a temperature of preferably from 10°C to 95°C, more preferably from 20°C to 75°C, even more preferably from 40°C to 70°C. The hydration can be performed according to any known methods. For example, hydration can be carried out by immersing a contact lens in a hydrating solution or dispersion for a time from 5 minutes to 72 hours.

In accordance with the invention, the hydrating solution comprises preferably from 100 ppm to 150000 ppm of silver and from 100 ppm to 150000 of a lubricant/wetting agent.

In accordance with the invention, a hydrogel contact lens is a lens comprising a hydrogel material, preferably a silicone hydrogel material. The lens can be prepared according to any methods known to a person skilled in the art. Preferably, the lens is prepared by curing in a mold a hydrogel lens-forming formulation. A "hydrogel lens-forming formulation" or "hydrogel lens-forming material" refers to a polymerizable composition which can be cured (i.e., polymerized and/or crosslinked) thermally or actinically to obtain a crosslinked/polymerized polymeric material. Lens-forming materials are well known to a person skilled in the art. Typically a lens forming material comprises polymerizable/crosslinkable components, for example, such as, monomers, macromers, prepolymers, or combinations thereof. A lens-forming material can further include other components, such as an initiator (e.g., a photoinitiator or a thermal initiator), a visibility tinting agent, UV-blocking agent, photosensitizers, antimicrobial agents (e.g., Ag-nanoparticles), lubricant/wetting agents (e.g., those described above), and the like.

In accordance with the present invention, a polymerizable fluid composition can be a solution or a solvent-free liquid or melt at a temperature below 60°C.

In accordance with a preferred embodiment of the invention, a silicone hydrogel lens-forming material comprises

at least one silicon-containing monomer or macromer or prepolymer, or can be any lens formulations for making silicone hydrogel contact lenses. Exemplary silicone hydrogel lens formulations include without limitation the formulations of lotrafilcon A, lotrafilcon B, etafilcon A, genfilcon A, lenefilcon A, polymacon, acquafilcon A, balafilcon, senofilcon A, and the like.

Where a polymerizable fluid composition is a solution, it can be prepared by dissolving at least one silicone-containing monomer, macromer or prepolymer and all other desired components in any suitable solvent known to a person skilled in the art. Examples of suitable solvents are water, alcohols, such as lower alkanols, for example ethanol or methanol, and furthermore carboxylic acid amides, such as dimethylformamide, dipolar aprotic solvents, such as dimethyl sulfoxide or methyl ethyl ketone, ketones, for example acetone or cyclohexanone, hydrocarbons, for example toluene, ethers, for example THF, dimethoxyethane or dioxane, and halogenated hydrocarbons, for example trichloroethane, and also mixtures of suitable solvents, for example mixtures of water with an alcohol, for example a water/ethanol or a water/methanol mixture.

Any known suitable silicone-containing monomers can be used in the present invention. In accordance with the invention, a monomer can be a silicone-containing vinylic monomer or a monomer with two thiol groups. Examples of silicone-containing monomers include, without limitation, methacryloxyalkylsiloxanes, 3-methacryloxy propylpentamethyldisiloxane, bis(methacryloxypropyl)tetramethyl-disiloxane, monomethacrylated polydimethylsiloxane, mercapto-terminated polydimethylsiloxane, N-[tris(trimethylsiloxy)silylpropyl]acrylamide, N-[tris(trimethylsiloxy)silylpropyl]methacrylamide, tris(pentamethyldisiloxyanyl)-3-methacrylatopropylsilane (T2), and tristrimethylsilyloxysilylpropyl methacrylate (THIS). A preferred siloxane-containing monomer is TRIS, which is referred to 3-methacryloxypropyltris(trimethylsiloxy) silane, and represented by CAS No. 17096-07-0. The term "TRIS" also includes dimers of 3-methacryloxypropyltris(trimethylsiloxy) silane. The silicone-containing monomer can also comprise one or more hydroxyl and/or amino groups.

Where the polymerization of the polymerizable dispersion is carried out based on thiol-ene step-growth radical polymerization, the silicone-containing monomer preferably comprises two thiol groups or one ene-containing group defined by any one of formula (I) - (III)

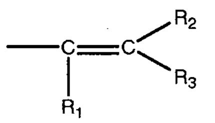

(I)

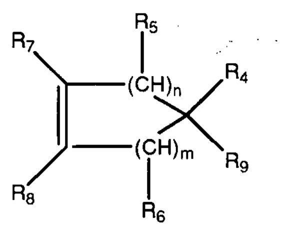

(II)

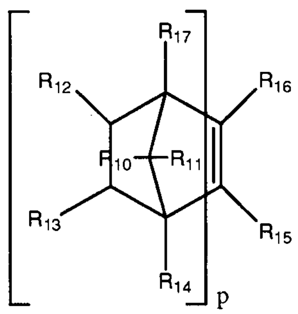

(III)

in which $R_1$ is hydrogen, or $C_1$-$C_{10}$ alkyl; $R_2$ and $R_3$ independent of each other are hydrogen, $C_1$-$C_{10}$ alkene divalent radical, $C_1$-$C_{10}$ alkyl, or $-(R_{18})_a-(X_1)_b-R_{19}$ in which $R_{18}$ is $C_1$-$C_{10}$ alkene divalent radical, $X_1$ is an ether linkage (-O-), a urethane linkage (-N), a urea linkage, an ester linkage, an amid linkage, or carbonyl, $R_{19}$ is hydrogen, a single bond, amino group, carboxylic group, hydroxyl group, carbonyl group, $C_1$-$C_{12}$ aminoalkyl group, $C_1$-$C_{18}$ alkylaminoalkyl group, $C_1$-$C_{18}$ carboxyalkyl group, $C_1$-$C_{18}$ hydroxyalkyl group, $C_1$-$C_{18}$ alkylalkoxy group, $C_1$-$C_{12}$ aminoalkoxy group, $C_1$-$C_{18}$ alkylaminoalkoxy group, $C_1$-$C_{18}$ carboxyalkoxy group, or $C_1$-$C_{18}$ hydroxyalkoxy group, a and b independent of each other is zero or 1, provided that only one of $R_2$ and $R_3$ is a divalent radical; $R_4$ - $R_9$, independent of each other, are

hydrogen, $C_1$-$C_{10}$ alkene divalent radical, $C_1$-$C_{10}$ alkyl, or $-(R_{18})_a-(X_1)_b-R_{19}$, optionally $R_4$ and $R_9$ are linked through an alkene divalent radical to form a cyclic ring, provided that at least one of $R_4$ - $R_9$ are divalent radicals; n and m independent of each other are integer number from 0 to 9, provided that the sum of n and m is an integer number from 2 to 9; $R_{10}$ - $R_{17}$, independent of each other, are hydrogen, $C_1$-$C_{10}$ alkene divalent radical, $C_1$-$C_{10}$ alkyl, or $-(R_{18})_a-(X_1)_b-R_{19}$, p is an integer number from 1 to 3, provided that only one or two of $R_{10}$ - $R_{17}$ are divalent radicals.

Any known suitable silicone-containing macromers can be used in the invention. In accordance with the invention, a macromer comprises one or more ethylenically unsaturated groups and/or at least two thiol groups, which can participate in free radical chain growth polymerization or thiol-ene step-growth radical polymerization. Preferably, a silicone-containing macromer is a siloxane-containing macromer. Any suitable siloxane-containing macromer with ethylenically unsaturated group(s) can be used to produce a silicone hydrogel material. A particularly preferred siloxane-containing macromer is selected from the group consisting of Macromer A, Macromer B, Macromer C, and Macromer D described in U.S. Patent No. 5,760,100. Macromers that contain two or more polymerizable groups (vinylic groups) can also serve as cross linkers. Di and triblock macromers consisting of polydimethylsiloxane and polyakyleneoxides could also be of utility. Such macromers could be mono or difunctionalized with acrylate, methacrylate or vinyl groups. For example one might use methacrylate end capped polyethyleneoxide-block-polydimethylsiloxane-block-polyethyleneoxide to enhance oxygen permeability.

Where the polymerization of the polymerizable dispersion is carried out based on thiol-ene step-growth radical polymerization, the silicone-containing macromer preferably comprises at least two thiol groups or one or more ene-containing groups defined by any one of formula (I) - (III) above.

In accordance with the invention, a prepolymer comprises one or more ethylenically unsaturated groups and/or at least two thiol groups, which can participate in free radical chain growth polymerization or thiol-ene step-growth radical polymerization. Examples of silicone-containing prepolymers include without limitation those disclosed in U.S. Patent Application Publication No. 2001-0037001 A1, U.S. Patent No. 6,039,913, and a co-pending U.S. Patent Application Serial No. 60/869,812 filed Dec. 13, 2006 (entitled "PRODUCTION OF OPHTHALMIC DEVICES BASED ON PHOTO-INDUCED STEP GROWTH POLYMERIZATION". Preferably, the prepolymers used in the invention are previously purified in a manner known *per se,* for example by precipitation with organic solvents, such as acetone, filtration and washing, extraction in a suitable solvent, dialysis or ultrafiltration, ultrafiltration being especially preferred. By means of that purification process the prepolymers can be obtained in extremely pure form, for example in the form of concentrated aqueous solutions that are free, or at least substantially free, from reaction products, such as salts, and from starting materials, such as, for example, non-polymeric constituents. The preferred purification process for the prepolymers used in the process according to the invention, ultrafiltration, can be carried out in a manner known *per se.* It is possible for the ultrafiltration to be carried out repeatedly, for example from two to ten times. Alternatively, the ultrafiltration can be carried out continuously until the selected degree of purity is attained. The selected degree of purity can in principle be as high as desired. A suitable measure for the degree of purity is, for example, the concentration of dissolved salts obtained as by-products, which can be determined simply in known manner.

Where the polymerization of the polymerizable dispersion is carried out based on thiol-ene step-growth radical polymerization, the silicone-containing prepolymer preferably comprises at least two thiol groups or one or more ene-containing groups defined by any one of formula (I) - (III) above.

In accordance with the present invention, a polymerizable fluid composition can also comprise a hydrophilic monomer. Nearly any hydrophilic monomer that can act as a plasticizer can be used in the fluid composition of the invention. Among the preferred hydrophilic vinylic monomers are N,N-dimethylacrylamide (DMA), 2-hydroxyethylmethacrylate (HEMA), hydroxyethyl acrylate, hydroxypropyl acrylate, hydroxypropyl methacrylate (HPMA), trimethylammonium 2-hydroxy propylmethacrylate hydrochloride, dimethylaminoethyl methacrylate (DMAEMA), dimethylaminoethylmethacrylamide, acrylamide, methacrylamide, allyl alcohol, vinylpyridine, glycerol methacrylate, N-(1,1dimethyl-3-oxobutyl)acrylamide, N-vinyl-2-pyrrolidone (NVP), acrylic acid, methacrylic acid, and N,N-dimethyacrylamide (DMA).

A polymerizable fluid composition can also comprises a hydrophobic monomer. By incorporating a certain amount of hydrophobic monomer in a polymerizable fluid composition, the mechanical properties (e.g., modulus of elasticity) of the resultant polymer may be improved.

In a preferred embodiment, a polymerizable fluid composition suitable for making a hydrogel contact lens will include (a) 20 to 40 weight percent of a siloxane-containing macromer, (b) 5 to 30 weight percent of a siloxane-containing monomer, and (c) 10 to 35 weight percent of a hydrophilic monomer. More preferably, the siloxane-containing monomer is TRIS.

In accordance with the present invention, a polymerizable fluid composition can further comprise various components, such as cross-linking agents, a chain transfer agent, initiator, UV-absorbers, inhibitors, fillers, visibility tinting agents (e.g., dyes, pigments, or mixtures thereof), antimicrobial agents (e.g., Ag-nanoparticles), lubricant/wetting agents (e.g., those described above), and the like, as known to a person skilled in the art.

In a preferred embodiment, the method of the invention further comprises a step of placing the hydrated lens in a packaging solution in a lens package, wherein the packaging solution comprises silver nanoparticles and a lubricant

or wetting agent.

Lens packages (or containers) are well known to a person skilled in the art for autoclaving and storing contact lenses. Any lens packages can be used in the invention. Preferably, a lens package is a blister package which comprises a base and a cover, wherein the cover is detachably sealed to the base, wherein the base includes a cavity for receiving a sterile packaging solution and the contact lens.

In accordance with the present invention, a packaging solution is ophthalmically compatible, meaning that a contact lens treated with the solution is generally suitable and safe for direct placement on the eye without rinsing, that is, the solution is safe and comfortable for contact with the eye via a contact lens that has been wetted with the solution. A packaging solutions of the invention may be any water-based solution that is used for the storage of contact lenses. A packaging solution of the invention can be a saline solution, a buffered solution, and deionized water. The preferred aqueous solution is saline solution containing the components described above.

Lenses are packaged in individual packages, sealed, and sterilized (e.g., by autoclave) prior to dispensing to users. A person skilled in the art will understand well how to seal and sterilize lens packages.

The invention, in another aspect, provides a method of making hydrogel contact lenses, the method of invention comprising the steps of: polymerizing a lens-forming formulation to form a hydrogel contact lens; optionally extracting unpolymerized polymerizable components in the lens-forming formulation by using an organic solvent; optionally contacting the polymerized hydrogel contact lens, which has been subjected to the extraction step, with a first aqueous solution containing silver nanoparticles and a lubricant or wetting agent, so as to replace, by water, the organic solvent entrapped in the hydrogel contact lens; and hydrating the polymerized hydrogel contact lens in a second aqueous solution containing silver nanoparticles and a lubricant or wetting agent, wherein the silver nanoparticles and the lubricant or wetting agent are adsorbed onto and/or entrapped in the hydrogel contact lens during the extraction step and/or the hydrating step.

Lens molds for making contact lenses are well known to a person skilled in the art and, for example, are employed in cast molding or spin casting. For example, a mold (for cast molding) generally comprises at least two mold sections (or portions) or mold halves, i.e. first and second mold halves. The first mold half defines a first molding (or optical) surface and the second mold half defines a second molding (or optical) surface. The first and second mold halves are configured to receive each other such that a lens forming cavity is formed between the first molding surface and the second molding surface. The molding surface of a mold half is the cavity-forming surface of the mold and in direct contact with lens-forming material.

Methods of manufacturing mold sections for cast-molding a contact lens are generally well known to those of ordinary skill in the art. The process of the present invention is not limited to any particular method of forming a mold. In fact, any method of forming a mold can be used in the present invention. The first and second mold halves can be formed through various techniques, such as injection molding or lathing. Examples of suitable processes for forming the mold halves are disclosed in U.S. Patent Nos. 4,444,711 to Schad; 4,460,534 to Boehm et al.; 5,843,346 to Morrill; and 5,894,002 to Boneberger et al.

Virtually all materials known in the art for making molds can be used to make molds for preparing contact lenses. For example, polymeric materials, such as polyethylene, polypropylene, polystyrene, PMMA, cyclic olefin copolymers (e.g., Topas® COC from Ticona GmbH of Frankfurt, Germany and Summit, New Jersey; Zeonex® and Zeonor® from Zeon Chemicals LP, Louisville, KY), or the like can be used. Other materials that allow UV light transmission could be used, such as quartz glass and sapphire.

In a preferred embodiment, when the polymerizable components in the fluid dispersion is composed essentially of prepolymers, reusable molds can be used. Examples of reusable molds made of quartz or glass are those disclosed in U.S. Patent No. 6,627,124. In this aspect, the fluid dispersion is poured into a mold consisting of two mold halves, the two mold halves not touching each other but having a thin gap of annular design arranged between them. The gap is connected to the mold cavity, so that excess prepolymer composition can flow into the gap. Instead of polypropylene molds that can be used only once, it is possible for reusable quartz, glass, sapphire molds to be used, since, following the production of a lens, these molds can be cleaned rapidly and effectively to remove unreacted materials and other residues, using water or a suitable solvent, and can be dried with air. Reusable molds can also be made of a cyclic olefin copolymer, such as for example, Topas® COC grade 8007-S10 (clear amorphous copolymer of ethylene and norbornene) from Ticona GmbH of Frankfurt, Germany and Summit, New Jersey, Zeonex® and Zeonor® from Zeon Chemicals LP, Louisville, KY. Because of the reusability of the mold halves, a relatively high outlay can be expended at the time of their production in order to obtain molds of extremely high precision and reproducibility. Since the mold halves do not touch each other in the region of the lens to be produced, i.e. the cavity or actual mold faces, damage as a result of contact is ruled out. This ensures a high service life of the molds, which, in particular, also ensures high reproducibility of the contact lenses to be produced and high fidelity to the lens design.

After the dispersion is dispensed into the mold, it is polymerized to produce a contact lens. Crosslinking and/or polymerizing may be initiated in the mold e.g. by means of actinic radiation, such as UV irradiation, ionizing radiation (e.g., gamma or X-ray irradiation). Where prepolymers of the invention are the polymerizable components in the fluid

composition, the mold containing the fluid composition can be exposed to a spatial limitation of actinic radiation to crosslink the prepolymers.

Above described various embodiments and preferred embodiments of hydrating solutions, hydrating techniques, Ag-nanoparticles and methods for preparing the same, chloride-treatment of Ag-nanoparticles, lubricant/wetting agents, lens-forming materials, packages, sealing and sterilization, and the others can be used in this aspect of the invention.

An exemplary aspect, provides a hydrogel contact lens.

Above described various embodiments and preferred embodiments of hydrogel material, hydrating solutions, hydrating techniques, Ag-nanoparticles and methods for preparing the same, chloride-treatment of Ag-nanoparticles, lubricant/wetting agents, lens-forming materials, packages, sealing and sterilization, and the others can be used in this aspect.

The silicone hydrogel contact lens has a wettable surface characterized by having an averaged water contact angle of 80 degrees or less, preferably 70 degrees or less, more preferably 60 degrees or less, even more preferably 50 degrees or less.

The silicone hydrogel contact lens has an oxygen permeability of at least 40 barrers, preferably at least 50 barrers, more preferably at least 65 barrers, even more preferably at least 80 barrers. An oxygen permeability is an apparent (directly measured when testing a sample with a thickness of 100 microns) oxygen permeability according to procedures described in Examples.

The silicone hydrogel contact lens can further have an elastic modulus of 1.5 MPa or less, preferably 1.2 MPa or less, more preferably 1.0 or less, even more preferably from 0.4 MPa to 1.0 MPa.

The silicone hydrogel contact lens further has an Ionoflux Diffusion Coefficient, D, of, preferably at least 1.5 x $10^{-6}$ $mm^2$/min, more preferably at least 2.6 x $10^{-6}$ $mm^2$/min, even more preferably at least 6.4 x $10^{-6}$ $mm^2$/min.

The silicone hydrogel contact lens further has a water content of preferably from 18% to 55%, more preferably from 20% to 38% by weight when fully hydrated. The water content of a silicone hydrogel contact lens can be measured according to Bulk Technique as disclosed in U.S. Patent No. 5,849,811.

The silicone hydrogel contact lens further comprises a leachable wetting agent.

The previous disclosure will enable one having ordinary skill in the art to practice the invention. In order to better enable the reader to understand specific embodiments and the advantages thereof, reference to the following examples is suggested.

**Example 1**

Unless otherwise stated, all chemicals are used as received. Oxygen and ion permeability measurements are carried out with lenses after extraction and plasma coating.

**Oxygen permeability measurements**. The oxygen permeability of a lens and oxygen transmissibility of a lens material is determined according to a technique similar to the one described in U.S. Patent No. 5,760,100 and in an article by Winterton et al., (The Cornea: Transactions of the World Congress on the Cornea 111, H.D. Cavanagh Ed., Raven Press: New York 1988, pp. 273-280),. Oxygen fluxes (J) are measured at 34°C in a wet cell (i.e., gas streams are maintained at about 100% relative humidity) using a Dk1000 instrument (available from Applied Design and Development Co., Norcross, GA), or similar analytical instrument. An air stream, having a known percentage of oxygen (e.g., 21%), is passed across one side of the lens at a rate of about 10 to 20 $cm^3$ /min., while a nitrogen stream is passed on the opposite side of the lens at a rate of about 10 to 20 $cm^3$ /min. A sample is equilibrated in a test media (i.e., saline or distilled water) at the prescribed test temperature for at least 30 minutes prior to measurement but not more than 45 minutes. Any test media used as the overlayer is equilibrated at the prescribed test temperature for at least 30 minutes prior to measurement but not more than 45 minutes. The stir motor's speed is set to 1200±50 rpm, corresponding to an indicated setting of 400±15 on the stepper motor controller. The barometric pressure surrounding the system, $P_{measured}$, is measured. The thickness (t) of the lens in the area being exposed for testing is determined by measuring about 10 locations with a Mitotoya micrometer VL-50, or similar instrument, and averaging the measurements. The oxygen concentration in the nitrogen stream (i.e., oxygen which diffuses through the lens) is measured using the DK1000 instrument. The apparent oxygen permeability of the lens material, $Dk_{app}$, is determined from the following formula:

$$Dk_{app} = Jt/(P_{oxygen})$$

where J=oxygen flux [microliters $O_2$ /$cm^2$ -minute]

$$P_{oxygen} = (P_{measured} - P_{water}\ vapor) = (\%O_2 \text{ in air stream})\ [mm\ Hg] = \text{partial pressure of oxygen in the air stream}$$

$P_{measured}$ =barometric pressure (mm Hg)
$P_{water}$ vapor =0 mm Hg at 34 °C (in a dry cell) (mm Hg)
$P_{water}$ vapor =40 mm Hg at 34 °C (in a wet cell) (mm Hg)
t=average thickness of the lens over the exposed test area (mm)

where $Dk_{app}$ is expressed in units of barrers.

The oxygen transmissibility (Dk /t) of the material may be calculated by dividing the oxygen permeability ($Dk_{app}$) by the average thickness (t) of the lens.

**Ion Permeability Measurements.** The ion permeability of a lens is measured according to procedures described in U.S. Patent No. 5,760,100. The values of ion permeability reported in the following examples are relative ionoflux diffusion coefficients ($D/D_{ref}$) in reference to a lens material, Alsacon, as reference material. Alsacon has an ionoflux diffusion coefficient of $0.314X10^3$ $mm^2$/minute.

<u>Surface hydrophilicity (wettability) Tests</u>

Water contact angle on a contact lens is a general measure of the surface hydrophilicity (or wettability) of the contact lens. In particular, a low water contact angle corresponds to more hydrophilic surface. Average contact angles (Sessile Drop) of contact lenses are measured using a VCA 2500 XE contact angle measurement device from AST, Inc., located in Boston, Massachusetts. This equipment is capable of measuring advancing or receding contact angles or sessile (static) contact angles. The measurements are performed on fully hydrated contact lenses and immediately after blot-drying.

**Antimicrobial Activity Assay.**

Antimicrobial activity of a contact lens with or without silver nanoparticles in the lenses of the invention is assayed against Pseudomonas aeruginosa GSU # 3, which is isolated from a corneal ulcer. Bacterial cells of Pseudomonas aeruginosa GSU # 3 are stored in a lyophilized state. Bacteria are grown on a Tryptic Soy agar slant for 18 hours at 37 °C. The cells are harvested by centrifugation and washed twice with sterile, Delbeco's phosphate buffered saline ("PBS"). Bacterial cells are suspended in PBS and adjusted to Optical Density of $10^8$ colony forming units or "cfu". The cell suspension is serially diluted to $10^3$ cfu/mJ.

Lenses having a silver in them are tested against the control lenses (i.e., without a silver). 200μl of from about $5x10^3$ to $1x10^4$ cfu/ml of Pseudomonas aeruginosa GSU #3 is placed on the surface of each lens. Incubate at 25 °C for 24 hours. Aspirate 50 μl out of the lens, serially dilute and plate out on agar plates to determine the microbial load of each lens. At 24 hours, colony counts are taken.

**Example 2**

**Synthesis of Macromer.**

51.5g (50 mmol) of the perfluoropolyether Fomblin® ZDOL (from Ausimont S.p.A, Milan) having a mean molecular weight of 1030 g/mol and containing 1.96meq/g of hydroxyl groups according to end-group titration is introduced into a three-neck flask together with 50mg of dibutyltin dilaurate. The flask contents are evacuated to about 20 mbar with stirring and subsequently decompressed with argon. This operation is repeated twice. 22.2g (0.1 mol) of freshly distilled isophorone diisocyanate kept under argon are subsequently added in a counterstream of argon. The temperature in the flask is kept below 30 °C by cooling with a waterbath. After stirring overnight at room temperature, the reaction is complete. Isocyanate titration gives an NCO content of 1.40 meq/g (theory: 1.35 meq/g).

202g of the α,ω-hydroxypropyl-terminated polydimethylsiloxane KF-6001 from Shin-Etsu having a mean molecular weight of 2000g/mol (1.00meq/g of hydroxyl groups according to titration) are introduced into a flask. The flask contents are evacuated to approximately 0.1 mbar and decompressed with argon. This operation is repeated twice. The degassed siloxane is dissolved in 202ml of freshly distilled toluene kept under argon, and 100mg of dibutyltin dilaurate (DBTDL) are added. After complete homogenization of the solution, all the perfluoropolyether reacted with isophorone diisocyanate (IPDI) is added under argon. After stirring overnight at room temperature, the reaction is complete. The solvent is stripped off under a high vacuum at room temperature. Microtitration shows 0.36meq/g of hydroxyl groups (theory 0.37meq/g).

13.78g (88.9mmol) of 2-isocyanatoethyl methacrylate (IEM) are added under argon to 247g of the α,α-hydroxypropyl-terminated polysiloxane-perfluoropolyether-polysiloxane three-block copolymer (a three-block copolymer on stoichiometric average, but other block lengths are also present). The mixture is stirred at room temperature for three days. Microtitration then no longer shows any isocyanate groups (detection limit 0.01 meq/g). 0.34meq/g of methacryl

groups is found (theory 0.34meq/g).

The macromer prepared in this way is completely colorless and clear. It can be stored in air at room temperature for several months in the absence of light without any change in molecular weight.

**Example 3**

**Preparation of stabilized silver nanoparticle solutions.**

Stabilized silver nanoparticle solutions are prepared by mixing an aqueous stabilizer (for example, polyacrylic acid (PAA), or polyethylenimine (PEI), or polyvinyl pyrrolidone (PVP), etc) and silver nitrate solution. Then this mixture solution of stabilizer and silver nitrate is added slowly into a solution containing a reducing agent. Some examples of reducing agents are sodium borohydride ($NaBH_4$) or ascorbic acid (also known as vitamin C, VC). Solutions of various silver concentrations and pHs can be prepared. For examples, the silver concentration can vary from 0.1 mM to 100mM; pH can be adjusted from 1.0 to 10.0. The molar ratio of the stabilizer to silver can also vary from 0.5/1 to 10/1. The ratio of silver to the reducing agent can also be adjusted, for example, from 4/1 to 1/10. Different molecular weight for PAA, PEI or PVP can be used.

As an example, a PAA stabilized silver nanoparticle solution is prepared as following: 0.759g sodium borohydride ($NaBH_4$, 98%, from J.T. Baker) is weighed and added into 1 L of ultra pure water (UPW). The solution is stirred for 30min using a magnetic stirring bar. 1.152g polyacrylic acid (PAA, Mw 90k, 25% aqueous solution, from Polysciences, Inc) is weighed and mixed into 20mL of ultra pure water (UPW). 0.34g silver nitrate ($AgNO_3$, 99.995%, from Aldrich) is weighed and mixed into 40mL of ultra pure water. Pour 40mL of $AgNO_3$ solution into the 20mL of PAA solution and allow the solution to be mixed for a minimum of 15 minutes. Remove 60mL of the $NaBH_4$ solution from the 1 liter $NaBH_4$ solution before adding the PAA-$AgNO_3$ mixture solution. Drip the PAA-$AgNO_3$ mixture solution slowly into the $NaBH_4$ solution. It is observed that the color of the solution is changed form clear to yellow due to formation of silver nanoparticles. This will lead to a PAA stabilized silver nanoparticle solution using $NaBH_4$ as reducing agent. This solution is called PAA-AgNP ($NaBH_4$) solution. Adjust the pH using nitric acid to desired pH (e.g. 1.5 or 2.0). Filter the solution before use. In this example, the final silver concentration is 2mM. The final concentration of PAA is 4 mM, calculated based on the molecular weight of acrylic acid (the repeating unit of PAA). The solution is then identified as PAA-AgNP (4mM-2mM) ($NaBH_4$) or PAA-AgNP -$NaBH_4$ solution.

Similarly, PAA stabilized silver nanoparticle solution using VC as reducing agent can be prepared. This solution is called PAA-AgNP (VC) or PAA-AgNP-VC solution. As a further example, PEI stabilized silver nanoparticle solution using $NaBH_4$ or VC as reducing agent can be prepared and is called PEI-AgNP ($NaBH_4$) or PEI-AgNP (VC) solution.

**Example 4**

**Hydration of dry Night & Day™ (CIBA Vision) (ND) lenses in PAA-AgNP-$NaBH_4$** solution.

Dry ND lenses are used directly after plasma coating. Or dry ND lenses can be obtained by taking finished lenses from commercial polypropylene packaging and equilibrated in fresh UPW 3 times for 20minutes ( total 1 hr) and then dried in a vacuum oven (normally at 40 °C for at least 2 hours). The dry lenses are hydrated by dipping into the PAA stabilized silver nanoparticle (PAA-AgNP, 0.4mM-0.2mM-solution) for a desired period of time (e.g., 1 hour). Then the lenses are rinsed in fresh UPW 3 times for 3 minutes each. Lenses are packaged in PBS and autoclaved.

**Example 5**

**Hydration of dry Night & Day™ (CIBA Vision) (ND) lenses in PAA-AgNP solution.**

The dry ND lenses described in Example 3 are hydrated by dipping into the PAA stabilized silver nanoparticle (PAA-AgNP-$NaBH_4$, 0.25mM-0.5mM-0.25mM, pH2.0) solution for certain time (e.g. 1 hour). Then the lenses are rinsed in water for about 30 seconds. Note in this example that not all the silver ions were reduced by $NaBH_4$ because the ratio of Ag to $NaBH_4$ is 2 to 1. Therefore, the lenses are immersed again in a 10mM VC solution to further reduce the silver ions to silver nanoparticles. After rinsed with water (3 times, 3 minutes each), lenses are packaged in PBS and autoclaved.

The antibacterial activity of the lenses are evaluated per assay described in Example 1, no bacteria are recovered. In other words, this lens exhibited almost ~ 100% reduction of bacteria under the conditions tested.

**Example 6**

**Hydration of dry Night & Day™ (CIBA Vision) (ND) lenses in PAA-AgNP-VC solution.**

Dry ND lenses described in Example 3 are hydrated by dipping into the PAA stabilized silver nanoparticle (PAA-AgNP-VC, 0.8mM-0.2mM-0.1 mM) solution for certain time (e.g. 1 hour). Then the lenses are rinsed in water for about 30 seconds. Note in this example that not all the silver ions were reduced by VC because the ratio of Ag to VC is 2 to 1. Therefore, the lenses are immersed again in a 10mM VC solution to further reduce the silver ions to silver nanoparticles. After rinsed with water (3 times, 3 minutes each), lenses are packaged in PBS and autoclaved.

The antibacterial activity of the lenses are evaluated per assay described in Example 1, no bacteria are recovered. In other words, this lens exhibited almost ~100% reduction of bacteria under the conditions tested.

**Example 7**

**Hydration of dry Night & Day™ (CIBA Vision) (ND) lenses in PAA-$AgNO_3$ solution.**

Dry ND lenses described in Example 3 are hydrated by dipping into the PAA-$AgNO_3$ mixture solution (PAA-$AgNO_3$, 5mM-5mM, pH2.5) solution for certain time (e.g. 1 hour). Then the lenses are rinsed in water for about 30 seconds. The lenses are then immersed again in a 10mM VC solution to reduce the silver ions to silver nanoparticles. After rinsed with water (3 times, 3 minutes each), lenses are packaged in PBS and autoclaved.

The antibacterial activity of the lenses are evaluated per assay described in Example 1, no bacteria are recovered. In other words, this lens exhibited almost ~100% reduction of bacteria under the conditions tested.

**Example 8**

**Hydration of dry Night & Day (ND) lenses in multiple silver nanoparticle solutions.**

Dry ND lenses described in Example 3 are hydrated in multiple silver nanoparticle solutions according to different approaches as following:

(1) Lenses are first hydrated in PAA-AgNP-$NaBH_4$ (0.4mM-0.2mM-2mM, pH2.0) solution for 1 hour, followed by a water rinsing step, then followed by a dipping in PEI-AgNP-VC (0.4mM-0.2mM-0.1 mM. pH4.2) solution for 10 minutes, followed by a water rinse, then again by a dipping in PAA-AgNP-$NaBH_4$ (0.4mM-0.2mM-2mM, pH2.0) solution for 10 minutes. After rinsed with water, lenses are packaged in PBS and autoclaved.

(2) Lenses are first hydrated in PAA-AgNP-$NaBH_4$ (0.4mM-0.2mM-2mM, pH2.0) solution for 1 hour, followed by a water rinsing step, then followed by a dipping in PEI-AgNP-VC (0.4mM-0.2mM-0.1mM. pH4.2) solution for 10 minutes, followed by a water rinse, then again by a dipping in PAA-AgNP-$NaBH_4$ (0.4mM-0.2mM-2mM, pH2.0) solution for 10 minutes, followed by a water rinsing step, then followed by a dipping in PEI-AgNP-VC (0.4mM-0.2mM-0.1 mM. pH4.2) solution for 10 minutes, followed by a water rinse, then again by a dipping in PAA-AgNP-$NaBH_4$ (0.4mM-0.2mM-2mM, pH2.0) solution for 10 minutes. After rinsed with water, lenses are packaged in PBS and autoclaved.

(3) Lenses are first hydrated in PAA-$AgNO_3$ (5mM-5mM, pH2.0) solution for 1 hour, followed by a water rinsing step, then followed by a dipping in PVP-AgNP-$NaBH_4$ (0.15mM-0.1mM-0.2mM. pH2.5) solution for 10 minutes. The molecular weight of PVP used in this example is 55000. Followed by a water rinse, the lenses are again dipped in PAA-AgNP-$NaBH_4$(1mM-1mM-2mM, pH2.0) solution for 10 minutes. After rinsed with water, lenses are packaged in PBS and autoclaved.

The antibacterial activity of the lenses from these hydration approaches are evaluated per assay described in Example 1, no bacteria are recovered. In other words, these lenses exhibited almost ~100% reduction of bacteria under the conditions tested.

**Example 9**

**Hydration (or one dip coating) of wet ND lenses.**

ND lenses directly from commercial polypropylene packaging are used. First, the lenses are equilibrated in fresh UPW 3 times for 20 minutes (total 1 hr). Then the wet lenses are dipped into the PAA stabilized silver nanoparticle (PAA-AgNP, 0.4mM-0.2mM, or 4mM-2mM) solution for certain time (e.g. 1 hour). Then the lenses are rinsed in fresh UPW 3 times for 3 minutes each. Lenses are packaged in glass vials with 2mL of PBS and autoclaved.

## Claims

1. A method of making hydrogel contact lenses, comprising the steps of hydrating a dry hydrogel contact lens in a hydrating solution, **characterized in that** the hydrating solution comprises silver nanoparticles and a lubricant or wetting agent, wherein the silver nanoparticles and the lubricant or wetting agent are adsorbed onto and/or entrapped in the hydrogel contact lens during hydrating of the dry hydrogel contact lens in the hydrating solution, wherein the silver nanoparticles are prepared in the presence of one or more stabilizers selected from the group consisting of a polyanionic material, a polycationic material, a polyvinyl alcohol (PVA), a polyvinylpyrrolidone (PVP), a copolymer of N-vinylpyrrolidone with one or more vinylic monomers, and combinations thereof.

2. The method of claim 1, wherein the hydrating solution comprises a lubricant or wetting agent selected from the group consisting of a polyvinyl alcohol, a polyamide, a polyimide, a polylactone, a polyvinylpyrrolidone, a copolymer of vinylpyrrolidone with one or more vinylic monomers, an alkylated polyvinylpyrrolidone, a homopolymer of acrylamide, a homopolymer of methacrylamide, a copolymer of acrylamide with one or more hydrophilic vinylic monomers, a copolymer of methacrylamide with one or more hydrophilic vinylic monomers, a polyethylene oxide, a polyoxyethylene derivative, poly 2-ethyl oxazoline, a heparin polysaccharide, a polysaccharide, and a mixture thereof.

3. The method of claim 1, wherein the hydrating solution comprises at least one polyvinylpyrrolidone with a molecular weight of 60-K or higher and at least one polyvinylpyrrolidone with a molecular weight PVP of lower than 60-K.

4. The method of claim 1, wherein the hydrating solution comprises at least one polyvinylpyrrolidone and at least one copolymer of vinylpyrrolidone with one or more vinylic monomers.

5. The method of claim 1, wherein the hydrating solution comprises at least one polyvinylpyrrolidone and at least one alkylated polyvinylpyrrolidone.

6. The method of claim 1, wherein the step of hydrating the dry hydrogel contact lens is performed at a temperature of from 10°C to 95°C.

7. The method of claim 1, wherein the method further comprises a step of placing the hydrated lens in a packaging solution in a lens package, wherein the packaging solution comprises: silver nanoparticles and a lubricant or wetting agent as defined in claim 1.

8. The method of claim 1, wherein the hydrogel contact lens is a silicone hydrogel contact lens.

9. The method according to any one of claims 1 to 8, further comprising the steps of:

   (1) polymerizing a lens-forming formulation to form a hydrogel contact lens;
   (2) hydrating the polymerized hydrogel contact lens in an aqueous solution containing silver nanoparticles and a lubricant or wetting agent as defined in claims 1-8.

10. The method according to claim 9, further comprising, between the steps (1) and (2), the steps of: extracting unpolymerized polymerizable components in the lens-forming formulation by using an organic solvent; and contacting the polymerized hydrogel contact lens, which has been subjected to the extraction step, with an aqueous solution containing silver nanoparticles and a lubricant or wetting agent, so as to replace, by water, the organic solvent entrapped in the hydrogel contact lens.

11. The method according to any one of claims 9 to 10, wherein the method further comprises a step of placing the hydrated lens in a packaging solution in a lens package, wherein the packaging solution comprises: silver nanoparticles and a lubricant or wetting agent.

## Patentansprüche

1. Ein Verfahren zur Herstellung von Hydrogelkontaktlinsen, umfassend die Schritte des Hydratisierens einer trockenen Hydrogelkontaktlinse in einer Hydratisierungslösung, **dadurch gekennzeichnet, dass** die Hydratisierungslösung Silbernanopartikel und ein Schmiermittel oder Netzmittel umfasst, worin die Silbernanopartikel und das Schmiermittel oder Netzmittel während des Hydratisierens der trockenen Hydrogelkontaktlinse in der Hydratisierungslösung an

die Hydrogel-kontaktlinse adsorbiert und/oder in diese eingelagert werden, worin die Silbernanopartikel in Anwesenheit von einem oder mehreren Stabilisatoren hergestellt werden, der(die) ausgewählt ist(sind) aus der Gruppe, die aus einem polyanionischen Material, einem polykationischen Material, einem Polyvinylalkohol (PVA), einem Polyvinylpyrrolidon (PVP), einem Copolymer von N-Vinylpyrrolidon mit einem oder mehreren vinylischen Monomeren, und Kombinationen davon besteht.

**2.** Das Verfahren von Anspruch 1, worin die Hydratisierungslösung ein Schmiermittel oder Netzmittel umfasst, das ausgewählt ist aus der Gruppe, die aus einem Polyvinylalkohol, einem Polyamid, einem Polyimid, einem Polylacton, einem Polyvinylpyrrolidon, einem Copolymer von Vinylpyrrolidon mit einem oder mehreren vinylischen Monomeren, einem alkylierten Polyvinylpyrrolidon, einem Homopolymer von Acrylamid, einem Homopolymer von Methacrylamid, einem Copolymer von Acrylamid mit einem oder mehreren hydrophilen vinylischen Monomeren, einem Copolymer von Methacrylamid mit einem oder mehreren hydrophilen vinylischen Monomeren, einem Polyethylenoxid, einem Polyoxyethylenderivat, Poly-2-ethyloxazolin, einem Heparinpolysaccharid, einem Polysaccharid, und einer Mischung davon besteht.

**3.** Das Verfahren von Anspruch 1, worin die Hydratisierungslösung mindestens ein Polyvinylpyrrolidon mit einem Molekulargewicht von 60-K oder höher und mindestens ein Polyvinylpyrrolidon mit einem Molekulargewicht PVP von weniger als 60-K umfasst.

**4.** Das Verfahren von Anspruch 1, worin die Hydratisierungslösung mindestens ein Polyvinylpyrrolidon und mindestens ein Copolymer von Vinylpyrrolidon mit einem oder mehreren vinylischen Monomeren umfasst.

**5.** Das Verfahren von Anspruch 1, worin die Hydratisierungslösung mindestens ein Polyvinylpyrrolidon und mindestens ein alkyliertes Polyvinylpyrrolidon umfasst.

**6.** Das Verfahren von Anspruch 1, worin der Schritt des Hydratisierens der trockenen Hydrogelkontaktlinse bei einer Temperatur von 10°C bis 95°C durchgeführt wird.

**7.** Das Verfahren von Anspruch 1, worin das Verfahren des Weiteren einen Schritt des Einbringens der hydratisierten Linse in eine Verpackungslösung in einer Linsenpackung umfasst, worin die Verpackungslösung umfasst: Silbernanopartikel und ein Schmiermittel oder Netzmittel wie in Anspruch 1 definiert.

**8.** Das Verfahren von Anspruch 1, worin die Hydrogelkontaktlinse eine Silikonhydrogelkontaktlinse ist.

**9.** Das Verfahren gemäss irgend einem der Ansprüche 1 bis 8, umfassend des Weiteren die Schritte des:

(1) Polymerisierens einer linsenbildenden Formulierung, um eine Hydrogelkontaktlinse zu bilden;
(2) Hydratisierens der polymerisierten Hydrogelkontaktlinse in einer wässerigen Lösung, die Silbernanopartikel und ein Schmiermittel oder Netzmittel wie in Ansprüchen 1-8 definiert umfasst.

**10.** Das Verfahren gemäss Anspruch 9, umfassend des Weiteren, zwischen den Schritten (1) und (2), die Schritte des: Extrahierens von nicht polymerisierten polymerisierbaren Komponenten in der linsenbildenden Formulierung unter Verwendung eines organischen Lösungsmittels, und Inkontaktbringens der polymerisierten Hydrogelkontaktlinse, die dem Extraktionsschritt unterzogen worden ist, mit einer wässerigen Lösung, enthaltend Silbernanopartikel und ein Schmiermittel oder Netzmittel, um das in der Hydrogelkontaktlinse eingelagerte organische Lösungsmittel durch Wasser zu ersetzen.

**11.** Das Verfahren gemäss irgend einem der Ansprüche 9 bis 10, worin das Verfahren des Weiteren einen Schritt des Einbringens der hydratisierten Linse in eine Verpackungslösung in einer Linsenpackung umfasst, worin die Verpackungslösung umfasst: Silbernanopartikel und ein Schmiermittel oder Netzmittel.

## Revendications

**1.** Procédé de fabrication de lentilles de contact en hydrogel, comprenant les étapes d’hydratation d’une lentille de contact en hydrogel sèche dans une solution hydratante, **caractérisé en ce que** la solution hydratante comprenne des nanoparticules d’argent et un lubrifiant ou un agent mouillant, dans lequel les nanoparticules d’argent et le lubrifiant ou l’agent mouillant sont adsorbés sur et/ou emprisonnés dans la lentille de contact en hydrogel durant

l'hydratation de la lentille de contact en hydrogel sèche dans la solution hydratante, dans lequel les nanoparticules d'argent sont préparées en présence d'un ou de plusieurs agents stabilisants choisis dans le groupe constitué par un matériau polyanionique, un matériau polycationique, un alcool polyvinylique (PVA), une polyvinylpyrrolidone (PVP), un copolymère de N-vinylpyrrolidone avec un ou plusieurs monomères vinyliques, et leurs combinaisons.

2. Procédé selon la revendication 1, dans lequel la solution hydratante comprend un lubrifiant ou un agent mouillant choisi dans le groupe constitué par un alcool polyvinylique, un polyamide, un polyimide, une polylactone, une polyvinylpyrrolidone, un copolymère de vinylpyrrolidone avec un ou plusieurs monomères vinyliques, une polyvinylpyrrolidone alkylée, un homopolymère d'acrylamide, un homopolymère de méthacrylamide, un copolymère d'acrylamide avec un ou plusieurs monomères vinyliques hydrophiles, un copolymère de méthacrylamide avec un ou plusieurs monomères vinyliques hydrophiles, un oxyde de polyéthylène, un dérivé de polyoxyéthylène, une poly-2-éthyloxazoline, un polysaccharide de type héparine, un polysaccharide, et un mélange de ceux-ci.

3. Procédé selon la revendication 1, dans lequel la solution hydratante comprend au moins une polyvinylpyrrolidone ayant un poids moléculaire de 60-K ou plus et au moins une polyvinylpyrrolidone ayant un poids moléculaire PVP inférieur à 60-K.

4. Procédé selon la revendication 1, dans lequel la solution hydratante comprend au moins une polyvinylpyrrolidone et au moins un copolymère de vinylpyrrolidone avec un ou plusieurs monomères vinyliques.

5. Procédé selon la revendication 1, dans lequel la solution hydratante comprend au moins une polyvinylpyrrolidone et au moins une polyvinylpyrrolidone alkylée.

6. Procédé selon la revendication 1, dans lequel l'étape d'hydratation de la lentille de contact en hydrogel sèche est effectuée à une température de 10°C à 95°C.

7. Procédé selon la revendication 1, ledit procédé comprenant en outre une étape consistant à placer la lentille hydratée dans une solution de conditionnement dans un emballage de lentille, où la solution de conditionnement comprend: des nanoparticules d'argent et un lubrifiant ou un agent mouillant comme il est défini dans la revendication 1.

8. Procédé selon la revendication 1, dans lequel la lentille de contact en hydrogel est une lentille de contact en hydrogel de silicone.

9. Procédé selon l'une quelconque des revendications 1 à 8, comprenant en outre les étapes consistant à:

   (1) polymériser une formulation de formation de lentille pour former une lentille de contact en hydrogel;
   (2) hydrater la lentille de contact en hydrogel polymérisée dans une solution aqueuse contenant des nanoparticules d'argent et un lubrifiant ou un agent mouillant comme il est défini dans les revendications 1-8.

10. Procédé selon la revendication 9, comprenant en outre, entre les étapes (1) et (2), les étapes consistant à: extraire les composants polymérisables non polymérisés dans la formulation de formation de lentille en utilisant un solvant organique; et mettre la lentille de contact en hydrogel polymérisée, qui a été soumise à l'étape d'extraction, en contact avec une solution aqueuse contenant des nanoparticules d'argent et un lubrifiant ou un agent mouillant de manière à remplacer par de l'eau le solvant organique emprisonné dans la lentille de contact en hydrogel.

11. Procédé selon l'une quelconque des revendications 9 à 10, ledit procédé comprenant en outre une étape consistant à placer la lentille hydratée dans une solution de conditionnement dans un emballage de lentille, où la solution de conditionnement comprend: des nanoparticules d'argent et un lubrifiant ou un agent mouillant.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4472327 A
- US 5358688 A
- US 5536861 A
- EP 0604369 A
- EP 0947856 A2
- US 5515117 A
- US 5213801 A
- US 5328954 A
- US 20050013842 A1
- US 20050058844 A1
- US 20050008676 A1
- EP 1754494 A2
- US 20070037898 A
- US 86981206 P
- US 6627124 B
- US 4312575 A
- US 4632844 A
- US 6451871 B
- US 6719929 B
- US 6793973 B
- US 6811805 B
- US 6896926 B
- US 5760100 A
- US 20010037001 A1
- US 6039913 A
- US 4444711 A, Schad
- US 4460534 A, Boehm
- US 5843346 A, Morrill
- US 5894002 A, Boneberger
- US 5849811 A


### Non-patent literature cited in the description


- **CHALKLEY et al.** *Am. J. Ophthalmology,* 1966, vol. 61, 866-869
- **WINTERTON et al.** The Cornea: Transactions of the World Congress on the Cornea. Raven Press, 1988, vol. 11, 273-280